(19) Europäisches Patentamt
European Patent Office
Office européen des brevets

(11) Veröffentlichungsnummer: **0 533 213 B1**

(12) # EUROPÄISCHE PATENTSCHRIFT

(45) Veröffentlichungstag der Patentschrift: **21.09.94**

(51) Int. Cl.$^{5}$: **A61K 31/55**, A61K 47/10

(21) Anmeldenummer: **92119073.2**

(22) Anmeldetag: **03.01.90**

(60) Veröffentlichungsnummer der früheren Anmeldung nach Art. 76 EPÜ: **0 378 086**

(54) **Verwendung des Wirkstoffs Azelastin und Glycerinethern zur Bekämpfung von Psoriasis-Erkrankungen.**

(30) Priorität: **11.01.89 DE 3900607**

(43) Veröffentlichungstag der Anmeldung:
**24.03.93 Patentblatt 93/12**

(45) Bekanntmachung des Hinweises auf die Patenterteilung:
**21.09.94 Patentblatt 94/38**

(84) Benannte Vertragsstaaten:
**AT BE CH DE DK ES FR GB GR IT LI LU NL SE**

(56) Entgegenhaltungen:
**DE-A- 3 343 530**
**US-A- 3 840 606**

**ARZNEIMITTEL FORSCHUNG Bd. 31, Nr. 8, 1981, Seiten 1212 - 1215 T.TANIGAWA ET AL'effect of azelastine hydrochloride on vascular permeability inhypersensitivity reaction skin site in guinea pig'**

(73) Patentinhaber: **ASTA Medica Aktiengesellschaft**
**An der Pikardie 10**
**D-01277 Dresden (DE)**

(72) Erfinder: **Engel, Jürgen, Dr.**
**Erlenweg 3**
**W-8755 Alzenau (DE)**
Erfinder: **Mollière, Michael, Dr.**
**de Neufville 8**
**W-6000 Frankturt 70 (DE)**
Erfinder: **Szelenyi, Istvan, Dr.**
**Händelstrasse 32**
**W-8501 Schwaig (DE)**

EP 0 533 213 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Der Arzneimittelwirkstoff Azelastin (chemische Bezeichnung 4-(p-chlor-benzyl)-2-(hexahydro-1-methyl-1H-azepin-4-yl)-1(2H)-phthalazinon) ist ein Asthmaprophylaktikum und Antiallergikum (siehe deutsche Patentschrift 21 64 058).

Es wurde nun gefunden, daß Azelastin auch gegen Psoriasis und hiermit verwandte Erkrankungen wirksam ist.

Die Erfindung betrifft Mittel zur Bekämpfung von Psoriasis-Erkrankungen wie Psoriasis und psoriasisverwandten Erkrankungen.
Unter Psoriasis-Erkrankungen werden im Sinne der Erfindung Hauterkrankungen verstanden, die mit Hyperkeratosen einhergehen wie insbesondere Psoriasis.

Wirkung von Azelastin bei Psoriasis-Erkrankungen und hiermit verwandten Erkrankungen (beispielsweise Psoriasis) und Prüfung dieser Wirkung.

Mäuse (Durchschnittsgewicht 25 g) erhielten 7 Tage lang täglich 1 mg/kg Azelastin oral. Am 8. Tag wurde die obere Hautschicht mit Sandpapier entfernt. Durch diese mechanische Irritation und Entfernung der obersten Hautschicht entsteht eine akute Reaktion, die morphologische Ähnlichkeiten zur psoriatischen Reaktion aufweist. Außerdem erhöht sich der Leukotrien-Gehalt in der Dermis und Epidermis. Dies geht mit einer Erniedrigung der Prostaglandin-Konzentration einher. Auch diese Veränderungen sind für Psoriasis typisch. Durch die Vorbehandlung mit Azelastin fanden diese Veränderungen nicht statt. Azelastin normalisiert beziehungsweise erniedrigt sogar die dermale beziehungsweise epidermale Leukotrien-Konzentration und erhöht die Konzentration der Prostaglandine.

Zur Behandlung von Psoriasis werden zur Zeit hauptsächlich Corticosteroide eingesetzt. Demgegenüber hat das Azelastin nur geringe beziehungsweise zu vernachlässigende Nebenwirkungen und hemmt die Leukotrien-Synthese in der Haut auch nach systemischer Gabe; darüberhinaus tritt durch Azelastin auch eine Hemmung der durch PAF (platelet activating factor) induzierten Ödembildung ein.

Die pharmazeutischen Zubereitungen für die Anwendung bei Psoriasis-Erkrankungen und hiermit verwandten Erkrankungen am Menschen enthalten im allgemeinen zwischen 2 bis 16 vorzugsweise 4 bis 8 mg Azelastin.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsform kommen zum Beispiel in Frage: Ölige oder alkoholische beziehungsweise wässrige Lösungen sowie Suspensionen und Emulsionen.

Die Einzeldosis von Azelastin für die Anwendung bei Psoriasis-Erkrankungen und hiermit verwandten Erkrankungen kann beispielsweise liegen:
bei Arzneiformen zur lokalen Applikation auf die Haut und Schleimhäute (zum Beispiel in Form von Lösungen, Lotionen, Emulsionen, Salben und so weiter) zwischen 0,1 und 10 Gewichts%, vorzugsweise 0,7 - 5 Gewichts%, insbesondere 1 - 5 Gewichts%.
-(Die Dosen sind jeweils bezogen auf die freie Base)-

Wirkung von Azelastin bei Entzündungserkrankungen und Prüfung dieser Wirkung

Azelastin hemmt dosisabhängig nach lokaler Applikation die durch lokale Verabreichung von 12-0-Tetradecanoylphorbol-acetat (TPA) beziehungsweise Arachidonsäureinduzierte Ödembildung des Mäuseohrs, (Methode von Young et al.)

Azelastin weist darüberhinaus ganz allgemein eine gute entzündungshemmende Wirkung sowohl nach topischer (lokaler) als auch nach systemischer Verabreichung auf. Prüfung dieser Wirkung erfolgte beispielsweise am durch Carrageenin induzierten Pfotenödem der Ratte (Methode nach Mörsdorf et al., Arch. int. Pharmacodyn. 192 (1971) Seite 111) beziehungsweise am chemisch induzierten Mäuseohrödem (Methode von Young et al., Journal of Investigative Dermatology 80 (1983) Seite 48-52 sowie 82 (1984) Seite 367).

Bei dem Untersuchungsmodell des durch Carrageenin induzierten Pfotenödems der Ratte nach oraler Gabe (Mörsdorf et al. 1971) werden die Testsubstanzen 2 Stunden vor Gabe des Carrageenins mit Hilfe einer Schlundsonde oral (intragastral) verabreicht. Das Ausmaß der Zunahme der Pfotenvolumina wird eine Stunde später gemessen. Durch dieses Verfahren (Abkürzung der Einwirkzeit von den üblichen 3 Stunden auf eine Stunde) ist es möglich, die Wirkung der Prüfsubstanzen auf die sich rasch freisetzenden Mediatoren, wie Leukotriene, PAF (platelet activating factor) zu untersuchen.

Beispielsweise wird beim Arachidonsäure-induzierten Mäuseohrödem bei einer Dosis von 3 mg peroral/kg Körpergewicht Maus eine 28%ige Hemmung des Ödems und bei topischer Applikation bei einer

Dosis von 0,25 mg/Mäuseohr eine 41%ige Hemmung erzielt.

Am Rattenpfotenödem (induziert durch Carrageenin, Bestimmung der Pfotenvolumina nach einer Stunde) wird zum Beispiel bei einer peroralen Dosis von 3,9 mg/kg Ratte eine 50%ige Hemmung der Ödembildung erzielt. Die niedrigste, bereits wirksame Dosis in den oben angegebenen Tierversuchen ist beispielsweise 1 - 2 mg/kg oral beziehungsweise 10 mg/kg bei topischer Anwendung (10 mg/kg entsprechen etwa 0,25 $mg/cm^2$ Körperoberfläche).

Als allgemeiner Dosisbereich für die Wirkung (Tierversuche wie oben) kommt beispielsweise in Frage:

2,5 -80 mg/kg topisch, insbesondere 5 - 40 mg/kg.

Die Einzeldosis von Azelastin für die Anwendung bei Psoriasis kann beispielsweise liegen:

bei Arzneiformen zur lokalen Applikation auf die Haut und Schleimhäute (zum Beispiel in Form von Lösungen, Lotionen, Emulsionen, Salben, Klysmen und so weiter) zwischen 0,1 und 10 Gewichts%, vorzugsweise 0,7 - 5 Gewichts%, insbesondere 1 - 5 Gewichts%;

- (Die Dosen sind jeweils bezogen auf die freie Base) -

Die pharmazeutischen Zubereitungen für die Anwendung bei den zuvor angegebenen Indikationen enthalten im allgemeinen zwischen 2 bis 16 vorzugsweise 4 bis 8 mg Azelastin.

Die Verabreichung kann beispielsweise in Form von Tabletten, Kapsel, Pillen, Dragees, Zäpfchen, Einläufe, Salben, Klysmen, Gelees, Gelen, Cremes, Puder, Stäubepulver, Suspensionen (Aerosolen) oder in flüssiger Form erfolgen. Als flüssige Anwendungsformen kommen zum Beispiel in Frage:

Ölige oder alkoholische beziehungsweise wässrige Lösungen sowie Suspensionen und Emulsionen. Bevorzugte Anwendungsformen sind Tabletten, die zwischen 2 und 16 mg vorzugsweise 4 und 10 mg Azelastin enthalten. Beispielsweise können 3mal täglich 1 bis 2 Tabletten mit einem Gehalt von 4 bis 8 mg Azelastin empfohlen werden. Bei oraler Verabreichung ist die minimale tägliche Dosis beispielsweise 4 mg; die maximale tägliche Dosis bei oraler Verabreichung soll nicht über 24 mg liegen.

Die akute Toxizität von Azelastin an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 120 und 170 mg/kg.

-(Die Dosen sind jeweils bezogen auf die freie Base)-

Die pharmazeutischen Zusammensetzungen beziehungsweise Arzneimittel enthalten als Wirkstoff Azelastin oder seine physiologisch verträglichen Salze. Der Wirkstoff liegt gegebenenfalls in Mischung mit anderen pharmakologisch beziehungsweise pharmazeutisch wirksamen Stoffen vor. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 und ff.; H.v.Czetsch-Lindenwald, Hilfsstoffe für Pharmazie und angrenzende Gebiete; Pharm. Ind., Heft 2 (1961), Seite 72 und ff.; Dr. H.P. Fiedler, Lexikon der Hilfsstoffe für Pharmazie, Kosmetik und angrenzende Gebiete Cantor KG. Aulendorf in Württemberg 1981.

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Zur Herstellung von dermal anzuwendenden Zubereitungen kommen in Frage die vorgenannten Substanzen und streichfähige oder flüssige Kohlenwasserstoffe wie Vaselin oder Paraffin oder Gele aus Paraffinkohlenwasserstoffen und Polyethylen, Fette und Öle pflanzlichen oder tierischen Ursprungs, die zum Teil auch hydriert sein können oder synthetische Fette wie Glyceride der Fettsäuren $C_8$-$C_{18}$, sodann Bienenwachs, Cetylpalmitat, Wollwachs, Wollwachsalkohole;

Fettalkohole wie Cetylalkohol, Stearylalkohol, Polyethylenglykole vom Molekulargewicht 200 bis 20 000; flüssige Wachse wie Isopropylmyristat, Isopropylstearat, Äthyloleat; Emulgatoren wie Natrium-, Kalium-, Ammoniumsalze der Stearinsäure oder Palmitinsäure sowie Triethanolaminstearat, Alkalisalze der Ölsäure, Ricinolsäure, Salze von sulfurierten Fettalkoholen wie Natriumlaurylsulfat, Natriumcetylsulfat, Natriumstearylsulfat, Salze der Gallensäure, Sterole wie Cholesterol, Partialfettsäureester mehrwertiger Alkohole wie Ethylenglykolmonostearat, Glycerolmonostearat, Pentaerythritmonostearat, Partialfettsäureester des Sorbitans, Partialfettsäureester des Polyoxyethylensorbitans, Sorbitolether des Polyoxyethylens, Fettsäureester des Polyoxyethylens, Fettalkoholether des Polyoxyethylens, Fettsäureester der Saccharose, Fettsäureester

des Polyglycerols, Lecithin.

Als Antioxydantien kommen beispielsweise Natriummetabisulfit, Ascorbinsäure, Gallussäure, Gallussäurealkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80° C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan. Bei den parenteralen Zubereitungsformen handelt es sich insbesondere um sterile beziehungsweise sterilisierte Erzeugnisse.

Zur Behandlung der Psoriasis wird Azelastin lokal, zum Beispiel in Form von Lösungen, Tinkturen, Suspensionen, Emulsionen, Salben, Gelen, Cremes, Pasten, Lotionen oder Shampoos eingesetzt. Bevorzugt werden wasserfreie Zubereitungen verwendet, wodurch die gleichzeitige Anwendung von Salicylsäure und/oder Harnstoff ermöglicht wird. Derartige Zubereitungen, die durch den Zusatz von Tensiden auch abwaschbar gemacht werden können, sind beispielsweise in der Deutschen Offenlegungsschrift 36 03 859 beschrieben. Der Harnstoff kann entweder als Tensid-Harnstoff-Einschlußverbindung oder auch in freier Form vorliegen. Zubereitungen, die weder Harnstoff noch Salicylsäure enthalten, können selbstverständlich auch Verwendung finden.

Die Einsatzkonzentrationen an Azelastin betragen hierbei beispielsweise 0,1 bis 10 % (Gewicht/Gewicht), vorzugsweise 0,5 bis 8 %, insbesondere 1 bis 5 %. Die Einsatzkonzentrationen an Salicylsäure betragen beispielsweise 0,1 bis 10 %, vorzugsweise 0,2 bis 8 %, insbesondere 0,5 bis 5 %. Die Einsatzkonzentrationen an Harnstoff betragen beispielsweise 1 bis 20 %, vorzugsweise 3 bis 18 %, insbesondere 5 bis 15 %.

Für die topische Applikation, aber auch für die Zubereitung als Arzneimittel für andere Applikationsarten hat es sich beispielsweise als günstig herausgestellt, das Azelastin zusammen mit wenigstens einem Alkylglycerin mit 2 bis 12 Kohlenstoffatomen im Alkylrest, der in Form einer Ethergruppe an eine der primären oder sekundären OH-Gruppen des Glycerins gebunden vorliegen kann, einzusetzen. Derartige Alkylglycerine steigern beziehungsweise verbessern die Wirkung des Azelastins. Bevorzugt werden hierbei Alkylglycerine mit 3 bis 9 C-Atomen allein oder in Mischung verwendet.

Besonders günstige Wirkungen besitzt daher ein Arzneimittel, welches

a) Azelastin und

b) ein Alkylglycerin der allgemeinen Formel I

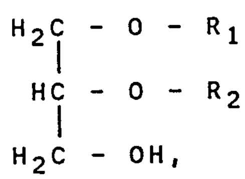

in welcher einer der Reste $R_1$ und $R_2$ eine Alkylgruppe mit 2 bis 12 C-Atomen und der andere Rest ein H-Atom bedeutet, enthält,

sowie gegebenenfalls weitere übliche pharmakologische Zusatz- und Verdünnungsmittel.

Vorzugsweise kommt ein Gemisch aus Wasser und einer Alkylglycerin-Mischung von Nonyl- beziehungsweise Octylglycerin, Hexyl- beziehungsweise Pentylglycerin und Propyl- beziehungsweise Ethylglycerin in Frage. Beispielsweise enthält eine entsprechende Formulierung für die topische Anwendung 1 bis 100 mg Azelastin pro ml Alkylglycerin der Formel I beziehungsweise einer entsprechenden Alkylglycerin-Mischung mit Wasser.

Eine derartige Mischung wird im folgenden auch als Kaskade bezeichnet.
Der Gehalt an Azelastin in mg/ml Kaskade wird durch einen nachgesetzten Index bezeichnet, derart, daß zum Beispiel ein Kaskadengemisch, welches 40 mg/ml Azelastin enthält, als Kaskade 40, ein Gemisch mit 60 mg Azelastin pro ml Kaskade als Kaskade 60 bezeichnet wird.

Die Herstellung der Alkylglycerine ist bekannt, beispielsweise aus der DE-OS 33 43 530.8. Beispielsweise werden Alkylglycerin-Wasser-Mischungen, welche zum Beispiel Nonylglycerin, Octylglycerin, Hexylglycerin, Pentylglycerin, Propylglycerin und Ethylglycerin enthalten, bevorzugt. Vorzugsweise enthalten solche wäßrigen Mischungen 3 der genannten Glycerinether, und zwar einen niederen (Ethyl, Propyl), einen mittleren (Pentyl, Hexyl) und einen höheren (Octyl, Nonyl), wobei die Gewichtsmenge an dem niederen Ether etwa so groß ist wie die Summe der Gewichtsmengen an den beiden anderen Glycerinethern. Die Wassermenge ist etwa gleich der Menge an dem niederen Glycerinether und beträgt beispielsweise die Hälfte der Gesamtmenge an den vorliegenden Glycerinethern. Beispiele für solche Glycerinether-Wasser-Mischungen sind im folgenden angeführt:

| | Wasser | | Glycerin-Propyl-ether | | Glycerin-Hexyl-ether | | Glycerin-Nonyl-ether |
|---|---|---|---|---|---|---|---|
| Gewichts-teile | 2 | : | 2 | : | 1 | : | 1 |

| | Wasser | | Glycerin-Ethyl-ether | | Glycerin-Pentyl-ether | | Glycerin-Octyl-ether |
|---|---|---|---|---|---|---|---|
| Gewichts-teile | 2 | : | 2 | : | 1 | : | 1 |

Zur topischen Applikation sind die Arzneimittel mit den Alkylglycerinen der Formel I in besonderem Maße geeignet. Um beispielsweise Psoriasis und hiermit verwandte Erkrankungen zu behandeln, werden die betroffenen Hautbezirke beispielsweise mit Kaskade 1 bis Kaskade 100 zwei- bis dreimal täglich eingerieben. Schädliche Nebenwirkungen konnten bisher nicht beobachtet werden.

Die topische Behandlung mit einer Azelastin-Kaskadenmischung läßt sich aber auch für die Behandlung von beispielsweise inneren entzündlichen Erkrankungen durch großflächiges Einreiben der Haut anwenden. Über die Resorption durch die Haut werden hierbei therapeutisch wirksame Blutspiegel erreicht. Ein Vorteil dieser Applikationsart liegt darin, daß die Kaskaden-Zubereitungen von der Haut problemlos toleriert werden.

Die Zubereitungsart des Azelastins in Form der Kaskade (zum Beispiel in Form der Lösungen Kaskade 1 bis Kaskade 100) eignet sich auch gut für die Herstellung von Suppositorien für die rektale Einführung. Auch hiermit lassen sich innere Entzündungserkrankungen gut behandeln.

Eine besonders günstige Trägermischung für das Azelastin besteht aus einer Mischung von etwa 4 Gewichtsteilen Wasser, 4 Gewichtsteilen Propylglycerin und je 2 Gewichtsteilen Hexylglycerin und Nonylglycerin.

Zur Herstellung der Arzneimittel, die das Azelastin in Gegenwart von einem Glycerinether der Formel I oder einer Mischung von solchen Glycerinethern der Formel I enthalten, wird das Azelastin beispielsweise mit 10000 bis 0,05, beispielsweise 1000 bis 5 oder 700 bis 7, vorzugsweise 30 bis 10 Gewichtsteilen (bezogen jeweils auf einen Gewichtsteil Azelastin) von mindestens einem Glycerinether der Formel I oder

einem Gemisch solcher Glycerinether sowie gegebenenfalls 10000 bis 0,05, beispielsweise 400 bis 2, vorzugsweise 20 bis 3 Gewichtsteilen Wasser (ebenfalls bezogen auf einen Gewichtstell Azelastin) verwendet. Diese Vermischung mit den Glycerinethern kann bei der Herstellung der entsprechenden Arzneimittel am Anfang erfolgen, aber gegebenenfalls auch in einem späteren Herstellungsstadium.

Beispiele

1. Beispiel für eine Formulierung zur topischen Behandlung von Psoriasis

Es handelt sich um eine Lösung von 8,8 % Azelastinhydrochlorid in einem Kaskade 0 genannten Lösungsmittelgemisch (Azelastin 8 %).

Herstellung der Kaskade 0

In einem geeigneten Behälter werden 1000 g Wasser, 1000 g Glycerin-1-n-propylether, 500 g Glycerin-1-n-hexylether und 500 g Glycerin-1-n-nonylether gemischt.

Herstellung der Azelastin-Lösung

In einem geeigneten Behälter werden etwa 2,5 Liter Kaskade 0 vorgelegt und 264 g Azelastinhydrochlorid in diesem Lösungsmittelgemisch aufgelöst. Anschließend wird mit Kaskade 0 auf 3 Liter aufgefüllt.
Die Dichte der Lösung beträgt 1,023 g/ml bei 22 °C. Diese Lösung wird unter aseptischen Bedingungen in ein steriles Vorlagegefäß durch einen Membranfilter mit einer Porengröße 0,2 µm sterilfiltriert und in sterile Tropfflaschen zu 10 ml abgefüllt. 1 ml Lösung enthält 80 mg Azelastin (entsprechend 88 mg Azelastin-HCl).

**Patentansprüche**

**1.** Verwendung von Azelastin und einem Alkylglycerin oder einer Mischung aus Alkylglycerinen der allgemeinen Formel I

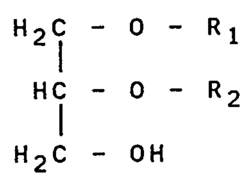

zur Herstellung eines Mittels zur Behandlung von Psoriasis-Erkrankungen.

**2.** Verfahren zur Herstellung eines Arzneimittels gemäß Anspruch 1, dadurch gekennzeichnet, daß man bei der Herstellung zusätzlich noch ein Alkylglycerin der Formel I

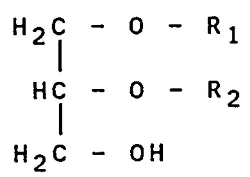

in der einer der Reste $R_1$ und $R_2$ eine Alkylgruppe mit 2 bis 12 C-Atomen und der andere Rest ein H-Atom bedeutet, oder ein Gemisch solcher Alkylglycerine sowie gegebenenfalls Wasser verwendet, wobei man 10000 bis 0,05 Gewichtsteile Alkylglycerin der Formel I beziehungsweise eines entsprechenden Alkylglyceringemisches und gegebenenfalls 10000 bis 0,05 Gewichtsteile Wasser, jeweils bezogen auf einen Gewichtstell Azelastin, verwendet.

## Claims

1. Use of azelastin and an alkylglycerol or a mixture of alkylglycerols of general formula I:

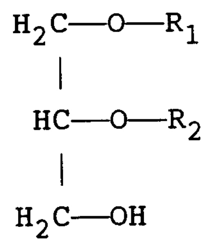

for the preparation of a composition for the treatment of psoriatic diseases.

2. A process for the preparation of a drug according to Claim 1, characterized in that an alkylglycerol of formula I:

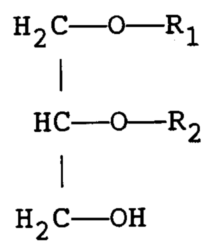

in which one of the radicals $R_1$ and $R_2$ is an alkyl group having 2 to 12 C atoms and the other radical is an H atom, or a mixture of such alkylglycerols, and optionally water are additionally used in the preparation, 10,000 to 0.05 parts by weight of alkylglycerol of formula I, or appropriate mixture of alkylglycerols, and optionally 10,000 to 0.05 parts by weight of water being used, based in each case on one part by weight of azelastin.

## Revendications

1. Utilisation d'azélastine et d'un alkylglycérol ou d'un mélange d'alkylglycérols de formule générale I

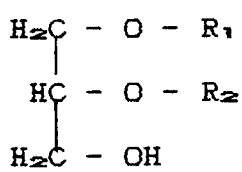

pour préparer un moyen de traitement du psoriasis.

2. Procédé de préparation d'un médicament selon la revendication 1, caractérisé en ce que l'on utilise de plus dans la préparation, encore un alkylglycérol de formule I

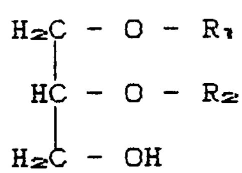

dans laquelle l'un des restes $R_1$ et $R_2$ représente un groupe alkyle ayant 2 à 12 atomes de carbone et l'autre reste représente un atome H, ou un mélange de tels alkylglycérols ainsi qu'éventuellement de l'eau, en utilisant 10000 à 0,05 parties en poids d'alkylglycérol de formule I ou d'un mélange d'alkylglycérols correspondants, et éventuellement 10000 à 0,05 parties en poids d'eau, chaque fois exprimées par rapport à une partie en poids d'azélastine.